# EUROPÄISCHE PATENTSCHRIFT

(11) **EP 0 663 815 B1**
(45) Veröffentlichungstag und Bekanntmachung des Hinweises auf die Patenterteilung: **18.03.1998**
(21) Anmeldenummer: 92924673.4
(22) Anmeldetag: 05.12.1992
(51) Int. Cl.: A61K 9/00, A61K 47/26

(54) **INHALATIONSPULVER UND VERFAHREN ZU IHRER HERSTELLUNG**
INHALATION POWDERS AND METHOD OF MANUFACTURING THEM
POUDRES A INHALER ET LEUR PROCEDE DE FABRICATION

(30) Priorität: 10.12.1991 DE 4140689
(43) Veröffentlichungstag der Anmeldung: 26.07.1995
(73) Patentinhaber: BOEHRINGER INGELHEIM INTERNATIONAL GmbH, 55218 Ingelheim am Rhein (DE); BOEHRINGER INGELHEIM KG, 55216 Ingelheim (DE)
(72) Erfinder: ARNOLD, Klaus, D-6507 Ingelheim (DE); GRASS, Peter, D-6507 Ingelheim (DE); KNECHT, Adolf, D-7800 Freiburg (DE); ROOS, Robert, D-6705 Ingelheim (DE); SLUKE, Gerhard, D-6507 Ingelheim (DE); THIEME, Herbert, D-6507 Ingelheim (DE); WENZEL, Joachim, D-6507 Ingelheim (DE)
(86) Internationale Anmeldenummer: EP9202814
(87) Internationale Veröffentlichungsnummer: WO9311746

(56) Entgegenhaltungen:
- FR-M- 8 142
- US-A- 2 533 065
- US-A- 4 009 280

## Beschreibung

Die Erfindung betrifft Inhalationspulver aus mikronisiertem Wirkstoff und Trägerstoffen mit bestimmten Anteilen an feinteiligen und gröberteiligen Partikeln sowie die Optimierung bzw. Steuerung des inhalierfähigen Wirkstoffanteils in den Inhalationspulvern.

Es ist bekannt, für die Praxis wichtige Eigenschaften von pulverförmigen Inhalationspräparaten zu verbessern, indem man das Medikament mit einer effektiven Teilchengröße von etwa 0,01 bis 10 µm mit einem wasserlöslichen Trägerstoff kombiniert, der eine effektive Teilchengröße zwischen 30 und 80 µm aufweist (DE-A-1792207).

Hilfsstoffe sind bei Inhalationspulvern vor allem dann nötig, wenn die Wirksamkeit des verarbeiteten Arzneistoffs sehr hoch ist, so daß pro Einzeldosis nur geringe Mengen benötigt werden. In diesem Fall ist die Verdünnung des Arzneistoffs ratsam, damit eine gute Dosiergenauigkeit erzielt wird.

Der meist relativ hohe Anteil an Hilfsstoff bestimmt im wesentlichen die Eigenschaften des Pulvers. Dies trifft insbesondere für das Fließverhalten zu. Je feiner ein Pulver ist, desto schlechter sind i.a. die Fließeigenschaften. Da gute Fließeigenschaften die Voraussetzung für eine gute Dosiergenauigkeit bei der Abfüllung einzelner Präparatedosen, etwa bei Herstellung von Kapseln zur Pulverinhalation auf üblichen Kapselmaschinen sind, darf der gewählte Hilfsstoff nicht zu fein sein.

Neben der Beeinflussung der Dosiergenauigkeit ist die Korngröße des Hilfsstoffes von großer Bedeutung für das Entleerungsverhalten von Kapseln in einem Inhalator bei der Anwendung. Es hat sich gezeigt, daß der inhalierbar ausgebrachte Wirkstoffanteil von einem grobkörnigen Hilfsstoff, wie ihn die DE-A 17 92 207 vorschlägt, negativ beeinflußt wird. Unter "inhalierfähig" werden solche Teilchen verstanden, die beim Inhalieren mit der Atemluft tief in die Verästelungen der Lunge transportiert werden. Die dazu erforderliche Teilchengröße liegt unter 10 µm, vorzugsweise unter 6 µm. Fließfähigkeit, Ausbringbarkeit und Dispergierbarkeit des Pulvers sind auch bei anderen Typen von Inhalationsgeräten von erheblicher Bedeutung, z.B. bei solchen, die die einzelne Dosis aus einem Vorrat mittels einer Meßkammer dosieren (z.B. gemäß US-A-4 570 630) oder in Vertiefungen einer Kreisscheibe enthalten (z.B. gemäß DE-A 36 25 685).

Es wurde nun gefunden, daß sich bei gleichzeitig guter Dosiergenauigkeit der inhalierfähige Anteil des Wirkstoffs von Inhalationspulvern in weiten Grenzen steuern läßt, wenn der zu inhalierfähigen Teilchen mikronisierte Wirkstoff mit geeigneten Mengen eines Gemisch aus einem oder mehreren physiologisch verträglichen Hilfsstoffen kombiniert wird, wobei der eine Anteil des Hilfstoffgemisches eine mittlere Teilchengröße unter etwa 10 µm, der andere eine mittlere Teilchengröße über etwa 20 µm aufweist, wobei die mittlere Teilchengröße im allgemeinen unter 150 µm, vorzugsweise unter 80 µm liegt.

Die Gewichtsverhältnisse von feinem und gröberem Hilfsstoff liegen zwischen 1:99 und 95:5, vorzugsweise zwischen 5:95 und 70:30, vor allem zwischen 10:90 und 50:50.

Da die Einzeldosis der meisten inhalativ angewendeten Arzneistoffe klein ist, ist ihr Anteil an der Mischung meist sehr gering, z.B. 0,01 bis 0,1 mg Wirkstoff pro ca. 5 mg Hilfstoffmischung. Die Menge der Zubereitung, die pro Inhaltionsvorgang verabreicht wird, kann innerhalb weiter Grenzen gewählt werden. Um dem Patienten nicht unnötig viel Hilfsstoff zuzuführen, wird der Fachmann bestrebt sein, die inhalierte Menge niedrig zu halten. Andererseits sind extrem geringe Mengen schlecht handhabbar und dosierbar. Dementsprechend wird die Menge an Zubereitung pro Anwendung zwischen etwa 1 und 20, vorzugsweise zwischen 2 und 10 mg liegen. Nach dem oben Gesagten ist jedoch eine Überschreitung der genannten Werte nach oben oder unten nicht grundsätzlich ausgeschlossen.

Wie oben erläutert, sind im allgemeinen die inhalativ verabreichten Wirkstoffe so stark wirksam, daß ihre Menge die Größe der Präparatemenge nicht entscheidend mitbestimmt. Vielmehr hat der Galeniker die Möglichkeit, durch Variation der Hilfsstoffmengen und des Teilchenspektrums den inhalierbar ausgebrachten Wirkstoffanteil zu steuern. Dies kann beispielsweise notwendig sein, wenn die gleiche Wirkung wie bei einer bereits klinisch geprüften oder im Handel befindlichen Darreichungsform erreicht werden soll.

Feinteiliger und gröberteiliger Hilfsstoff können aus dem chemisch gleichen oder aus chemisch verschiedenen Substanzen bestehen. Die Hilfsstoffmischungen können z.B. eine chemische Substanz als den feinen, eine andere als den gröberen Hilfsstoff enthalten. Aber auch die jeweiligen feinen und gröberen Hilfsstoffe können in sich Mischungen aus verschiedenen Substanzen sein. Neben den genannten Wirk- und Hilfsstoffen können die erfindungsgemäßen Zubereitungen in untergeordneter Menge weitere Zusätze enthalten, z.B. Geschmackskorrigenzien.

Pharmazeutisch geeignete und physiologisch unbedenkliche Hilfsstoffe für inhalative Zwecke sind bekannt. Beispiele sind Monosaccharide (etwa Glucose, Arabinose); Disaccharide (etwa Lactose, Saccharose, Maltose); Polysaccharide (etwa Dextrane); Polyalkohole (etwa Sorbit, Mannit, Xylit); Salze (etwa Natriumchlorid, Calciumcarbonat) oder auch Mischungen dieser Hilfsstoffe miteinander. Bevorzugt sind Lactose und Glucose.

Die nachstehenden Beispiele zeigen, wie unterschiedliche Verhältnisse von Hilfsstoffen den inhalierbar ausgebrachten Wirkstoffanteil beeinflussen. Als Hilfsstoffe wurden Glucose mit einer durchschnittlichen Teilchengröße von 35 µm (G I) und von 5 µm (G II) bzw. 8 µm (G III) verwendet. Die Mischung der Hilfsstoffe mit dem Wirkstoff wurde in 5 mg-Portionen in übliche Kapseln für die Pulverinhalation gefüllt und aus diesen mittels eines Geräts gemäß DE-A-33 45 722 ausgebracht.

Im Beispiel 1 enthielt die Mischung pro Kapsel 0,1 mg Fenoterol mit einer mittleren Teilchengröße von <6 µm und im Beispiel 2 0,04 mg Ipratropiumbromid mit einer mittleren Teilchengröße <6 µm.

"W" ist der inhalierbar ausgebrachte Wirkstoffanteil in Prozent der in der Mischung enthaltenen Menge.

| Beispiel 1 | | |
|---|---|---|
| G I [%] | G II [%] | W [%] |
| 100 | 0 | 14,4 |
| 96 | 4 | 21,7 |
| 84 | 16 | 31,0 |

| Beispiel 2 | | |
|---|---|---|
| G I [%] | G III [%] | W [%] |
| 100 | 0 | 15,1 |
| 95 | 5 | 15,7 |
| 90 | 10 | 23,0 |
| 75 | 25 | 33,4 |
| 50 | 50 | 38,3 |

## Patentansprüche

1. Inhalationspulver aus mikronisiertem Wirkstoff und mindestens einem physiologisch unbedenklichen Hilfsstoff, der gröbere Anteile mit einer mittleren Teilchengröße von 20 µm oder mehr und feinere Anteile mit einer mittleren Teilchengröße von 10 µm oder Weniger enthält, wobei das Gewichtsverhältnis der feineren zu den gröberen Hilfsstoffanteilen zwischen 1 : 99 und 95 : 5 liegt, und ggf. weiteren Hilfsstoffen, insbesondere Geschmackskorrigenzien mit der Maßgabe, daß daß in einer Mischung, welche aus 20 Gewichtsteilen kristalliner Laktose (1-10 µm mit mindestens 50 Gew.% im Intervall 2-6 µm) als feinerer Hilsstoffanteil, 19.9 Gewichtsteilen kristalliner Laktose (32-63 µm) als grober Hilfsstoffanteil, als auch einem Wirkstoff besteht, der Wirkstoff nicht 0.1 Gewichtsteile Isoprenalinsulfat von 1-10 µm mit mindestens 50 Gew.-% im Intervall 2-6 µm sein darf.

2. Inhalationspulver nach Anspruch 1, dadurch gekennzeichnet, daß das Gewichtsverhältnis der feineren zu den gröberen Hilfsstoffanteilen zwischen 5 : 95 und 70 : 30 liegt.

3. Inhalationspulver nach Anspruch 1 oder 2 dadurch gekennzeichnet daß das Gewichtsverhältnis der feineren zu den gröberen Hilfsstoffanteilen zwischen 10 : 90 und 50 : 50 liegt.

4. Inhalationspulver nach Anspruch 1, 2 oder 3, dadurch gekennzeichnet, daß der gröbere und der feinere Hilfsstoff aus derselben chemischen Substanz bestehen.

5. Inhalationspulver nach Anspruch 1, 2 oder 3, dadurch gekennzeichnet, daß der gröbere und der feinerteilige Hilfsstoff aus verschiedenen chemischen Substanzen bestehen.

6. Inhalationspulver nach Anspruch 1 bis 3 oder 5, dadurch gekennzeichnet, daß der gröbere und/oder der feinerteilige Hilfsstoff je für sich Mischungen darstellen.

7. Inhalationspulver nach Anspruch 1 bis 6, dadurch gekennzeichnet, daß die physiologisch unbedenklichen Hilfsstoffe zur Gruppe der Monosaccharide, Disaccharide, Polysaccharide, Polyalkohole oder anorganischen Salze gehören.

8. Verfahren zur Steuerung bzw. Optimierung des inhalationsfähigen Anteils von Inhalationspulvern, dadurch gekennzeichnet, daß man den auf eine Teilchengröße unter 10, vorzugsweise unter 6 µm mikronisierten Wirkstoff mit Hilfsstoff mischt, wobei der Hilfsstoff aus einem Anteil mit einer mittleren Teilchengröße < 10 µm und einem Anteil mit einer mittleren Teilchengröße > 20 µm besteht, und wobei das Gewichtsverhältnis der Teilchensorten zwischen 1 : 99 und 95 : 5, vorzugsweise zwischen 5 : 95 und 70 : 30, insbesondere zwischen 10 : 90 und 50 : 50 liegt, mit der Maßgabe, daß in einer Mischung, welche aus 20 Gewichtsteilen kristalliner Laktose (1-10 µm mit mindestens 50 Gew.% im Intervall 2-6 µ) als feinerer Hilfsstoffanteil, 19.9 Gewichtsteilen kristalliner Laktose (32-63 µm) als grober Hilfsstoffanteil, als auch einem Wirkstoff besteht, der Wirkstoff nicht 0.1 Gewichtsteile Isoprenalinsulfat von 1-10 µm mit mindestens 50 Gew.-% im Intervall 2-6 µm sein darf.

9. Verfahren zur Herstellung eines Aerosols für die Inhalation, dadurch gekennzeichnet, daß man ein Inhalationspulver nach Anspruch 1 bis 7 nach Anspruch 8 hergestelltes Inhalationspulver mittels eines für die Pulverinhalation geeigneten Geräts der vom Patienten eingeatmeten Luft beimischt.

## Claims

1. Powder for inhalation consisting of micronised active substance and at least one physiologically acceptable excipient which contains coarser fractions having an average particle size of 20 µm or more and finer fractions with an average particle size of 10 µm or less, the ratio by weight of the finer to coarser fractions of excipient being between 1 : 99 and 95 : 5, and optionally other excipients, particularly flavour correctors, with the proviso that, in a mixture consisting of 20 parts by weight of crystalline lactose (1-10 µm with at least 50% by weight in the range from 2-6 µm) as the finer fraction of excipient, 19.9 parts by weight of crystalline lactose (32-63 µm) as the coarser fraction of excipient and an active substance, the active substance must not be 0.1 parts by weight of isoprenaline sulphate of 1-10 µm with at least 50% by weight in the range from 2-6 µm.

2. Powder for inhalation according to claim 1, characterised in that the weight ratio of the finer excipient fractions to the coarser excipient fractions is between 5:95 and 70:30.

3. Powder for inhalation according to claim 1 or 2, characterised in that the weight ratio of the finer excipient fractions to the coarser excipient fractions is between 10:90 and 50:50.

4. Powder for inhalation according to claim 1, 2 or 3, characterised in that the coarser and finer excipients consist of the same chemical substance.

5. Powder for inhalation according to claim 1, 2 or 3, characterised in that the coarser and finer excipients consist of different chemical substances.

6. Powder for inhalation according to claim 1 to 3 or 5, characterised in that the coarser and/or the finer excipients each consist of a mixture.

7. Powder for inhalation according to claims 1 to 6, characterised in that the physiologically acceptable excipients belong to the group comprising the monosaccharides, disaccharides, polysaccharides, polyalcohols or inorganic salts.

8. Process for controlling or optimising the inhalable content of powders for inhalation, characterised in that the active substance micronised to a particle size of less than 10 and preferably less than 6 µm is mixed with excipient, the excipient consisting of a fraction having an average particle size of <10 µm and a fraction having an average particle size >20 µm, the weight ratio between the types of particles being between 1:99 and 95:5, preferably between 5:95 and 70:30, more especially between 10:90 and 50:50, with the proviso that, in a mixture consisting of 20 parts by weight of crystalline lactose (1-10 µm with at least 50% by weight in the range from 2-6 µ) as the finer fraction of excipient, 19.9 parts by weight of crystalline lactose (32-63 µm) as the coarser fraction of excipient and an active substance, the active substance must not be 0.1 parts by weight of isoprenaline sulphate of 1-10 µm with at least 50% by weight in the range from 2-6 µm.

9. Process for preparing an aerosol for inhalation, characterised in that a powder for inhalation according to claims 1 to 7 or a powder for inhalation prepared according to claim 8 is mixed with the air breathed in by the patient, by means of an apparatus suitable for powder inhalation.

## Revendications

1. Poudre à inhaler constituée par un principe actif micronisé et au moins un adjuvant physiologiquement irréprochable qui contient des fractions plus grossières ayant une granulométrie moyenne de 20 µm ou plus et des fractions plus fines ayant une granulométrie moyenne de 10 µm ou moins, le rapport massique des fractions plus fines aux fractions plus grossières d'adjuvant étant situé entre 1 :99 et 95 :5, et éventuellement d'autres adjuvants, en particulier des agents de correction du goût, avec la condition que, dans un mélange qui consiste en 20 parties en masse de lactose cristallin (1-10 µm avec au moins 50% en masse dans l'intervalle 2-6 µm) comme fraction d'adjuvant plus fine, 19,9 parties en masse de lactose cristallin (32-63 µm) comme fraction d'adjuvant plus grossière, et aussi en un principe actif, le principe actif ne peut pas être 0,1 partie en masse de sulfate d'isoprénaline de 1-10 µm avec au moins 50% en masse dans l'intervalle 2-6 µm.

2. Poudre à inhaler selon la revendication 1 caractérisée en ce que le rapport massique des fractions d'adjuvant plus fines aux fractions d'adjuvant plus grossières est situé entre 5 :95 et 70 :30.

3. Poudre à inhaler selon la revendication 1 ou 2 caractérisée en ce que le rapport massique des fractions d'adjuvant plus fines aux fractions d'adjuvant plus grossières est situé entre 10 :90 et 50 :50.

4. Poudre à inhaler selon la revendication 1, 2 ou 3 caractérisée en ce que l'adjuvant plus grossier et l'adjuvant plus fin consistent en la même substance chimique.

5. Poudre à inhaler selon la revendication 1, 2 ou 3 caractérisée en ce que l'adjuvant plus grossier et l'adjuvant plus fin consistent en des substances chimiques différentes.

6. Poudre à inhaler selon les revendications 1 à 3 ou 5 caractérisée en ce que l'adjuvant plus grossier et/ou l'adjuvant plus fin représentent en soi des mélanges.

7. Poudre à inhaler selon les revendications 1 à 6 caractérisée en ce que les adjuvants physiologiquement irréprochables appartiennent au groupe des monosaccharides, des disaccharides, des polysaccharides, des polyalcools ou des sels inorganiques.

8. Procédé pour commander ou optimiser la fraction inhalable de poudres à inhaler caractérisé en ce que l'on mélange le principe actif micronisé à une granulométrie inférieure à 10, de préférence inférieure à 6 µm, avec un adjuvant, l'adjuvant consistant en une fraction ayant une granulométrie moyenne < 10 µm et en une fraction ayant une granulométrie moyenne > 20 µm, et le rapport massique des sortes de particules étant situé entre 1 :99 et 95 :5, de préférence entre 5 :95 et 70 :30, en particulier entre 10 :90 et 50 :50, avec la condition que, dans un mélange qui consiste en 20 parties en masse de lactose cristallin (1-10 µm avec au moins 50% en masse dans l'intervalle 2-6 µm) comme fraction d'adjuvant plus fine, 19,9 parties en masse de lactose cristallin (32-63 µm) comme fraction d'adjuvant plus grossière, et aussi en un principe actif, le principe actif ne peut pas être 0,1 partie en masse de sulfate d'isoprénaline de 1-10 µm avec au moins 50% en masse dans l'intervalle 2-6 µm.

9. Procédé pour préparer un aérosol pour l'inhalation caractérisé en ce que l'on mélange une poudre à inhaler selon les revendications 1 à 7 ou une poudre à inhaler préparée selon la revendication 8 avec l'air inspiré par le patient au moyen d'un appareil convenant à l'inhalation de poudres.
